# EUROPÄISCHE PATENTSCHRIFT

(11) **EP 1 803 408 B1**
(45) Veröffentlichungstag und Bekanntmachung des Hinweises auf die Patenterteilung: **24.12.2008**
(21) Anmeldenummer: 06024000.9
(22) Anmeldetag: 20.11.2006
(51) Int. Cl.: A61B 18/12, A61B 17/00, A61B 17/34, A61B 18/14, A61B 17/04

(54) **Vorrichtung zum Verschließen des persistierenden Foramen Ovale**
Patent foramen ovale closure device
Dispositif pour la fermeture du foramen ovale patent

(30) Priorität: 28.12.2005 DE 102005062658
(43) Veröffentlichungstag der Anmeldung: 04.07.2007
(73) Patentinhaber: Osypka, Peter, 79618 Rheinfelden-Herten (DE)
(72) Erfinder: Osypka, Peter, 79618 Rheinfelden-Herten (DE)
(74) Vertreter: Maucher, Wolfgang

(56) Entgegenhaltungen:
- WO-A-20/04043272
- WO-A-20/04100812
- US-A1- 2004 243 122
- US-A1- 2004 254 572
- US-A1- 2005 033 288
- US-A1- 2005 119 675

## Beschreibung

Die Erfindung betrifft eine Vorrichtung zum Verschließen einer durch zwei sich überlappende Gewebelappen im Herzen gebildeten Öffnung gemäß dem Oberbegriff von Patentanspruch 1.

Eine derartige Vorrichtung ist aus US 2005/0192654 A1 und dabei vor allem aus den Figuren 10A bis 10E und Figur 14 bis 16 bekannt. Damit der Zugkatheter an dem in Zuführrichtung hinteren zweiten Gewebelappen angreifen kann, muss er zusammen mit einem radial gegenüber ihm vorstehenden Anker durch beide Gewebelappen hindurch bewegt werden, wofür innerhalb des ersten Zuführkatheters ein zweiter Zuführkatheter erforderlich ist, der gleichzeitig auch einen Anschlag für den in Zuführrichtung vorderen oder ersten Gewebelappen trägt. Dieser zweite innere Zuführkatheter für den Zugkatheter muss an seinem den Zugkatheter mit Anker zunächst in sich aufnehmenden Ende als Hohlnadel mit scharfem Rand ausgebildet sein, um durch die beiden Gewebelappen hindurchgestochen werden zu können.

Auch bei scharf geschliffener Stirnseite dieses hohlnadelartigen Endes besteht jedoch die Gefahr, dass die Ränder der Öffnung in der Trennwand bzw. die sie begrenzenden Gewebelappen aufgrund ihrer geringen Festigkeit ausweichen, so dass die geschliffenen Ränder der Hohlnadel bzw. des hohlnadelartigen zweiten Zuführkatheters gar nicht in die Gewebelappen eindringen und somit der Zugkatheter nicht in Gebrauchsstellung gelangt, so dass das Platzieren des Ankerelements verhindert wird.

Aus US 2004/0243122 A1, Fig. 8, geht ebenfalls eine vergleichbare Vorrichtung der eingangs definierten Art hervor, die die Merkmale des Oberbegriffs des Anspruchs 1 aufweist. Jedoch besteht dabei auch das Problem, dass der Zugkatheter durch beide Gewebelappen hindurch bewegt werden muss und die Gefahr besteht, dass die Gewebelappen aufgrund ihrer geringen Festigkeit ausweichen.

Aus US 2004/0254572 A1 ist eine Vorrichtung anderer Gattung, nämlich ein elektrochirurgisches Instrument mit einer als Schraubwendel ausgebildeten Elektrode bekannt, um Tumore durch Ablation zu zerstören. Die Schraubwendel ermöglicht es, das Instrument mit dem Tumor fest zu verbinden, so dass eine stiftförmige Gegenelektrode mit hoher Sicherheit und Präzision in den Tumor eingestochen werden kann.

Aus WO 2004/100812 A1 ist eine andere Ablationsvorrichtung mit zwei konzentrischen Schraubwendelelektroden bekannt, die beide in das zu zerstörende Gewebe einzudrehen sind. Auch dabei handelt es sich um eine Vorrichtung anderer Gattung.

US 2005/0033288 A1 offenbart eine elektrochirurgische Vorrichtung anderer Gattung zum Durchlöchern der Herztrennwand, wobei ein Vakuumkanal verwendet wird, um die Herztrennwand bei der Operation zu stabilisieren.

Es besteht deshalb die Aufgabe, eine Vorrichtung der eingangs genannten Art zu schaffen, mit welcher bei transvenöser Zuführung eine zwischen sich überlappenden Gewebelappen oder Hautfalten in einer Herztrennwand befindliche Öffnung verschlossen werden kann, wobei ein potentielles Ausweichen des oder der Gewebelappen trotz ihrer geringen Festigkeit beim Ansetzen der Elektrokoagulationsvorrichtung vermieden werden können soll. Außerdem soll die Vorrichtung eine präzise Kontrolle über die relative Position beider Gewebelappen ermöglichen, damit sich diese beim Koagulieren in enger Berührung miteinander befinden.

Zur Lösung dieser Aufgabe ist die eingangs definierte Vorrichtung mit der Einrichtung zum Verbinden der Gewebelappen dadurch gekennzeichnet, dass der erste Schraubkatether wenigstens eine am distalen Ende angeordnete, in den den Zuführkatheter in Gebrauchsstellung näherliegenden ersten Gewebelappen eindrehbare Schraubwendel aufweist und dass der zum Heranziehen des zweiten Gewebelappens an den ersten Gewebelappen dienende Zugkatheter als zweiter Schraubkatheter mit wenigstens einer Schraubwendel ausgebildet ist, die zusammen mit ihm durch den ersten Schraubkatheter hindurch verschiebbar ist und deren Schraubwendel oder Schraubwendeln in den den Zuführkatheter ablegenden zweiten Gewebelappen eindrehbar ist oder sind, oder dass als Zugkatheter ein Saugröhrchen oder -schlauch vorgesehen ist, der durch eine Punktionsöffnung in dem dem Zuführkatheter zugewandten ersten Gewebelappen bis zu dem diesen überlappenden zweiten Gewebelappen verschiebbar und mit seinem proximalen Ende an eine Unterdruckquelle anschließbar ist.

Mit dem ersten Schraubkatheter kann also der dem Zuführkatheter näherliegende erste Gewebelappen der zu verschließenden Öffnung im Herzen dadurch gegen ein Ausweichen bei weiteren Eingriffen gehindert werden, dass er mit einer Schraubwendel oder gegebenenfalls mit zwei oder mehr in Umfangsrichtung versetzten, gleichgroßen, gleichgerichteten und einem übereinstimmenden Steigungswinkel aufweisenden Schraubwendeln des ersten Schraubkatheters erfasst wird, so dass auf ihn bei einer Druckkraft eine entsprechende Gegen-Zugkraft über diesen ersten Schraubkatheter aufgebracht werden kann. Somit kann der erste Gewebelappen ohne Ausweichen durchquert und dadurch der zweite Gewebelappen erreicht werden, der mit Hilfe des Zugkatheters an den ersten Gewebelappen heranziehbar ist, wonach die beiden Gewebelappen durch eine lokale Koagulation mittels Hochfrequenzstrom gegebenenfalls an mehreren Stellen dauerhaft verbunden werden und endgültig zusammenwachsen können. Da die Katheter und Elektroden wieder entfernt werden können, bleibt bei diesem PFO-Verschluss in vorteilhafter Weise kein "Fremdmaterial" im Körper oder im Herzen zurück.

Da der zum Heranziehen des zweiten Gewebelappens an den ersten Gewebelappen dienende Zugkatheter auch als Schraubkatheter mit wenigstens einer Schraubwendel ausgebildet sein kann, die durch den ersten Schraubkatheter hindurch verschiebbar ist und deren Schraubwendel oder Schraubwendeln in den dem Zuführkatheter abliegenden zweiten Gewebelappen eindrehbar ist oder sind, kann mit diesem zweiten Schraubkatheter, der wiederum transvenös durch den ersten Schraubkatheter und damit auch durch den Zuführkatheter zugeführt werden kann, einen mechanische Verbindung zu dem zweiten Gewebelappen hergestellt werden, so dass dieser problemlos durch Zug an dem zweiten Schraubkatheter in Berührposition mit den ersten Gewebelappen gebracht werden kann, ohne dass ein Ausweichen eines der Gewebelappen zu befürchten ist.

Alternativ kann als Zugkatheter, wie erwähnt, ein Saugröhrchen oder -schlauch vorgesehen sein, der aus elektrisch leitendem Material besteht oder eine solche elektrische Leitung aufweist, so dass er an den Hochfrequenzgenerator anschließbar ist und die zweite Elektrode mit dem zweiten Pol für die Hochfrequenz-Koagulation bilden kann.

Günstig ist es dabei, wenn der erste Schraubkatheter als Hochfrequenzkatheter ausgebildet und mit einem Hochfrequenzspannungsgeber oder Hochfrequenz-Generator verbindbar ist. Weiterhin ist es dabei vorteilhaft, dass der zweite Schraubkatheter als Gegenelektrode ausgebildet und an einen Hochfrequenzspannungsgeber oder Hochfrequenz-Generator anschließbar ist. Bei einer solchen Ausführungsform bilden also die beiden Schraubkatheter die Elektroden oder Pole zum Applizieren des Hochfrequenz-Stroms für die dauerhafte Verbindung der beiden Gewebelappen, bevor diese endgültig und vollständig zusammenwachsen.

Da diese Vorrichtungsteile transvenös einführbar sind, ist der operative Aufwand für diesen - in der Fachsprache so genannten - PFO-Verschluss vergleichsweise gering.

Die Vorrichtung kann ein Punktionsinstrument und/oder einen Dilatator aufweisen, welches Instrument durch den Zuführkatheter und/oder durch den ersten Schraubkatheter hindurch vorschiebbar und zur Bildung einer Durchtrittsöffnung im inneren Bereich der Schraubwendel des ersten Schraubkatheters durch den ersten Gewebelappen hindurchstechbar ist. Es kann also mit Hilfe eines Punktionsinstruments oder Dilatators nach dem Einschrauben der Schraubwendel des ersten Schraubkatheters durch den von dieser Schraubwendel umschlossenen Bereich des ersten Gewebelappens hindurch eine Öffnung in dem ersten Gewebelappen angebracht werden, die das Zuführen des Zugkatheters, beispielsweise eines zweiten Schraubkatheters, zu dem in Zuführrichtung hinteren zweiten Gewebelappen erleichtert.

Bei Kombination einzelner oder mehrerer der vorbeschriebenen Merkmale und Maßnahmen ergibt sich also ein PFO-Verschluss mittels Hochfrequenzstrom-Erwärmung. Dabei kann der einem transvenös zuführbaren Zuführkatheter näherliegende erste Gewebelappen beziehungsweise eine Hautfalte in der Herztrennwand mittels der Schraubwendel des durch den Zuführkatheter in Gebrauchsstellung gebrachten ersten Schraubkatheters erfasst und fixiert werden. Danach kann dieser vordere beziehungsweise dem Zuführkatheter näherliegende erste Gewebelappen punktiert werden, wonach ein als zweiter Schraubkatheter geringen Querschnitts oder als Saugröhrchen oder - schlauch ausgebildeter Zugkatheter durch die punktierte und mit Hilfe des ersten Schraubkatheters gehaltene Hautfalte beziehungsweise den ersten Gewebelappen hindurch in den Bereich des hinteren zweiten Gewebelappens gebracht und dort durch Einschrauben oder mittels Unterdruck so verbunden wird, dass dieser zweite Gewebelappen durch Zug an dem Zugkatheter an den vorderen ersten Gewebelappen herangezogen werden kann.

Danach kann mit Hilfe von Hochfrequenzspannung mit Hilfe eines üblichen Hochfrequenzgenerators eine lokale Erwärmung der beiden Gewebelappen oder Hautfalten erfolgen, wodurch es zu einer mechanischen Verbindung beziehungsweise zu einem Verkleben der beiden Hautfalten kommt. Dadurch wird veranlasst, dass die beiden Gewebelappen oder Hautfalten zusammenwachsen und sich damit die PFO-Öffnung komplett verschließt. Die lokale Koagulation mittels Hochfrequenzstrom wird dabei je nach der Größe dieser Öffnung gegebenenfalls an mehreren Stellen durchgeführt.

Dabei ist es auch möglich, dass mehrere Zuführkatheter mit an einen Hochfrequenzgenerator anschließbarem Hochfrequenzkatheter und Gegenelektrode vorgesehen sind oder der Zuführkatheter mit Schraubkatheter und Hochfrequenzkatheter sowie Gegenelektrode mehrfach an benachbarten Stellen der Herztrennwand anwendbar ist.

Nachstehend sind Ausführungsbeispiele der Erfindung anhand der Zeichnung näher beschrieben. Es zeigt in zum Teil erheblich schematisierter Darstellung:

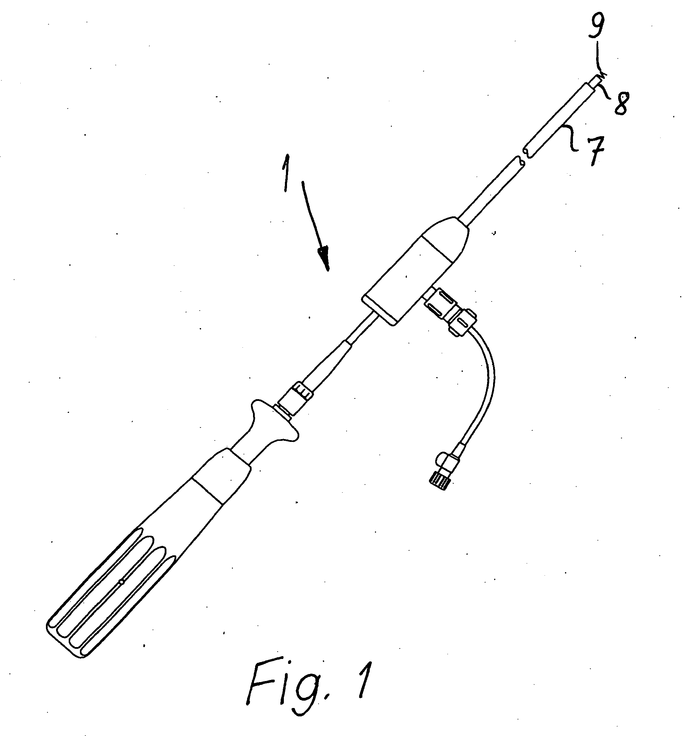

- Fig. 1: eine Seitenansicht einer erfindungsgemäßen Vorrichtung mit einem Zuführkatheter und mit einem darin angeordneten und durch ihn verschiebbaren ersten Schraubkatheter, dessen proximales Ende aus dem Führungskatheter nach hinten vorsteht und einen Haltegriff zum Verschieben und Verdrehen und Handhaben aufweist, während an dem distalen Ende dieses ersten Schraubkatheters eine Schraubwendel angeordnet ist, die in der dargestellten Position aus dem Zuführkatheter vorsteht,

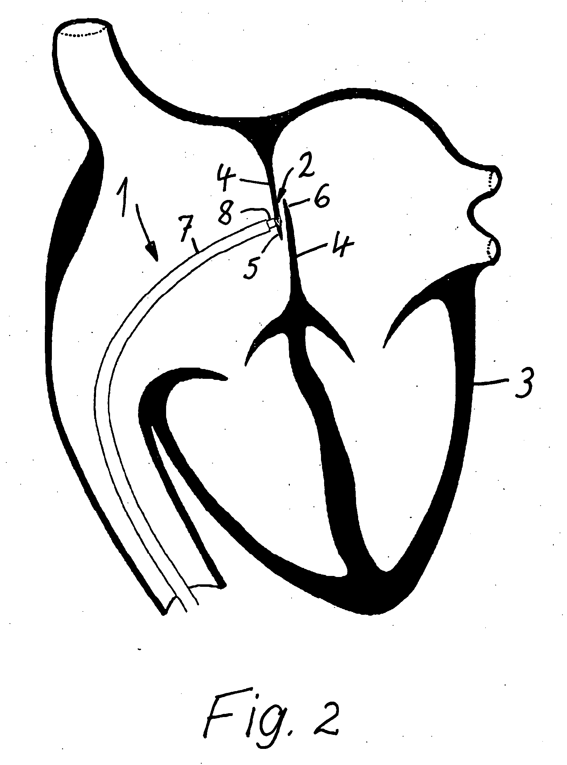

- Fig. 2: einen Schnitt durch ein Herz und durch den linken und rechten Vorhof, wobei in der Trennwand zwischen diesen beiden Vorhöfen eine Öffnung angeordnet ist, die durch zwei sich überlappende Gewebelappen oder Hautfalten dieser Trennwand begrenzt ist und wobei der Führungskatheter mit dem ersten Schraubkatheter transvenös in dem rechten Vorhof eingeführt und mit seiner Mündung der Trennwand derart angenähert ist, dass die Schraubwendel des Schraubkatheters in den dem Führungskatheter näherliegenden ersten Gewebelappen eindrehbar beziehungsweise in der zeichnerischen Darstellung bereits darin eingedreht ist und diesen ersten Gewebelappen über den Schraubkatheter gegen ein Ausweichen beim Einstechen eines weiteren Instruments, insbesondere eines Punktionsinstruments und/oder Dilatators hindert,

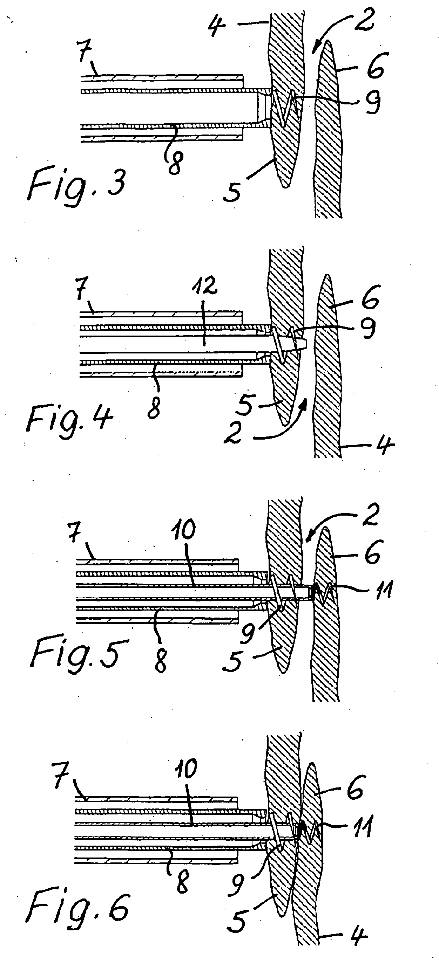

- Fig. 3: im vergrößertem Maßstab das distale Ende des Zuführkatheters und des durch diesen vorgeschobenen ersten Schraubkatheters in der Situation gemäß Fig.2, wobei das distale Ende des Zuführkatheters und des darin vorgeschobenen Schraubkatheters nach dem Einschrauben der Schraubwendel in den dem Zuführkatheter näherliegenden ersten Gewebelappen der Herztrennwand dargestellt sind,
- Fig. 4: eine der Fig. 3 entsprechende Darstellung nach dem Vorschieben eines Punktionsinstruments durch das Zentrum der Schraubwendel des ersten Schraubkatheters und durch den ersten Gewebelappen hindurch, wobei dieser während des Durchtritts des Punktionsinstruments durch die Schraubwendel und den Schraubkatheter entgegen der beim Durchstechen auftretenden Kraft zurückgehalten wird,
- Fig. 5: eine den Fig.3 und 4 entsprechende Darstellung nach dem Punktieren und Dilatieren sowie nach dem Einschieben eines zweiten Schraubkatheters durch den ersten Schraubkatheter und durch den dem Führungskatheter näherliegenden ersten Gewebelappen hindurch und nach dem Einschrauben seiner Schraubwendel in den in Einschubrichtung hinteren zweiten Gewebelappen, wobei an diesem zweiten Schraubkatheter eine Isolierung vorgesehen ist, so dass die beiden Schraubkatheter als an unterschiedliche Hochfrequenzpole eines Hochfrequenzgenerators anschließbare Elektroden dienen können,
- Fig. 6: eine der Fig. 5 entsprechende Darstellung nach dem Heranziehen des zweiten Gewebelappens mit Hilfe der zweiten Schraubelektrode in eine Berührposition mit dem ersten Gewebelappen, wonach durch lokale Koagulation beide Gewebelappen in dieser Position miteinander verbindbar sind, so dass sie danach zusammenwachsen,

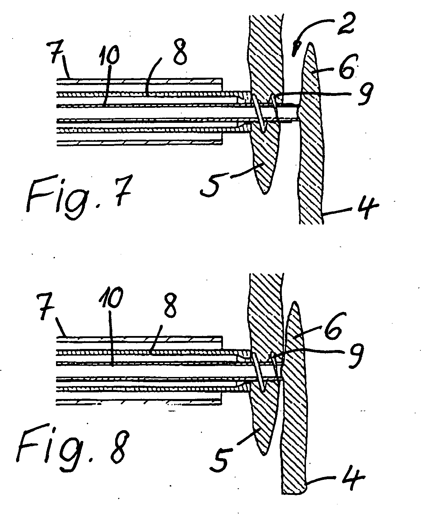

- Fig. 7: eine der Fig.5 entsprechende Darstellung, bei welcher aber nach dem Anbringen der ersten Schraubelektrode und der Punktion des von dieser erfassten Gewebelappens als Zugkatheter und zweite Elektrode nicht eine zweite Schraubwendel sondern ein Saugröhrchen oder Saugschlauch zu dem zweiten oder hinteren Gewebelappen vorgeschoben ist und diesen mittels an diesem Saugröhrchen angelegten Unterdruck erfasst, sowie
- Fig. 8: eine der Fig.6 entsprechende Darstellung nach dem Heranziehen des vom Führungskatheter aus gesehen hinteren zweiten Gewebelappens an den vorderen ersten Gewebelappen, indem auf das Saugröhrchen eine relative Zugkraft ausgeübt wird, wonach die beiden Gewebelappen mittels Hochfrequenz zusammengeklebt werden.

Eine im Ganzen mit 1 bezeichnete Vorrichtung, die auch als Bausatz bezeichnet oder angesehen werden kann, dient zum Verschließen einer Öffnung 2 in einer im Herzen 3 zwischen zwei Herzkammern, insbesondere zwischen dem rechten und dem linken Vorhof, befindlichen Trennwand 4, wobei diese Öffnung 2 durch zwei sich überlappende Gewebelappen 5 und 6 begrenzt ist.

Dabei erhalten in ihrer Funktion übereinstimmende Teile und Gegenstände auch bei unterschiedlicher Ausgestaltung übereinstimmende Bezugszahlen.

Die Vorrichtung 1 dient zur Herstellung eines PFO-Verschlusses und weist einen transvenös in das Herz 3 einführbaren Zuführkatheter 7 zum transvenösen Einführen einer noch näher zu beschreibenden Einrichtung zum Verbinden der Gewebelappen 5 und 6 miteinander auf. Diese Einrichtung zum Verbinden der beiden Gewebelappen 5 und 6 weist einen in dem Zuführkatheter 7 verschiebbaren und an dessen distalem Ende mit seinem distalen Ende ausschiebbaren ersten Schraubkatheter 8 mit einer an dessen distalem Ende angeordneten, in den dem Zuführkatheter 7 in Gebrauchsstellung näherliegenden ersten Gewebelappen 5 eindrehbaren Schraubwendel 9 und außerdem einen durch den dem Führungskatheter 7 näherliegenden ersten Gewebelappen 5 hindurch mit dem dahinterliegenden zweiten Gewebelappen 6 verbindbaren Zugkatheter 10 auf, der gemäß den Fig. 5 und 6 einerseits und gemäß den Fig.7 und 8 andererseits in noch zu beschreibender Weise unterschiedlich gestaltet sein kann. Dabei könnten auch mehrere, vorzugsweise zwei übereinstimmende, in Umfangsrichtung gegeneinander versetzte Schraubwendeln 9 vorgesehen sein, womit das Gewebe noch effektiver erfasst werden kann.

Mit Hilfe dieses Zugkatheters 10 kann der dem Führungskatheter 7 abgewandte hintere zweite Gewebelappen 6 gemäß den Fig. 6 und 8 an den ersten Gewebelappen 5 bis zur gegenseitigen Berührung herangezogen werden.

Für die gegenseitige Verbindung der beiden Gewebelappen 5 und 6 ist vorgesehen, dass ein mit dem ersten Gewebelappen 5 in Berührung befindlicher Hochfrequenzkatheter, nämlich der an einen Hochfrequenzgenerator anschließbare erste Schraubkatheter 8 einen ersten Pol, beispielsweise seine Schraubwendel, und der zum Heranziehen des zweiten Gewebelappens 6 dienende Zugkatheter 10 als Gegenelektrode mit einem weiteren Pol ausgebildet sind. Zwischen diesen beiden Elektroden oder Polen kann in nicht näher dargestellter Weise am proximalen Ende der Vorrichtung 1 eine Hochfrequenzspannung angelegt werden, damit mit Hilfe eines Hochfrequenzstroms die sich nunmehr berührenden Gewebelappen 5 und 6 koaguliert und lokal zusammengeklebt werden können, um ihr Zusammenwachsen zu veranlassen.

Die beiden Elektroden und Pole sind dabei in Gebrauchsstellung voneinander beabstandet und/oder gegeneinander isoliert, um einen Kurzschluss zu vermeiden.

Wie bereits erwähnt, ist dabei der erste Schraubkatheter 8 mit der Schraubwendel 9 als Hochfrequenzkatheter ausgebildet und kann mit einem Hochfrequenzspannungsgeber oder Hochfrequenzgenerator verbunden sein oder verbunden werden.

Der zum Heranziehen des zweiten Gewebelappens 6 an den ersten Gewebelappen 5 dienende Zugkatheter 10 ist im Ausführungsbeispiel gemäß Fig.5 und 6 als zweiter Schraubkatheter mit Schraubwendel 11 ausgebildet, die durch den ersten Schraubkatheter 8 und dessen Schraubwendel 9 hindurch vorschiebbar ist und in den dem Zuführkatheter 7 abliegenden zweiten Gewebelappen 6 eindrehbar ist, wie es in Fig.5 dargestellt ist. In diesem Falle ist der zweite Schraubkatheter mit der Schraubwendel 11 als Gegenelektrode ausgebildet und an einen Hochfrequenzspannungsgeber oder Hochfrequenzgenerator anschließbar, so dass zwei voneinander isolierte Elektroden und Pole für die gewünschte Koagulation zur Verfügung stehen. Auch dabei könnten zwei oder mehr jeweils miteinander übereinstimmende Schraubwendeln 11 vorgesehen sein.

Zu der Vorrichtung 1 gehört in beiden Ausführungsbeispielen auch ein Punktionsinstrument 12, dessen distales Ende als Dilatator ausgebildet ist und durch den Zuführkatheter 7 und den ersten Schraubkatheter 8 sowie dessen Schraubwendel 9 hindurch vorschiebbar und zur Bildung einer Durchtrittsöffnung im inneren Bereich dieser ersten Schraubwendel 9 in dem ersten Gewebelappen 5 dient. Man erkennt die Wirkungsweise des Punktionsinstruments 12 und der an seinem Ende befindlichen Verjüngung in Fig.4. Somit ist das Einführen und Einschrauben des zweiten Schraubkatheters mit seiner Schraubwendel 11 erleichtert, wobei während der Punktion und auch bei dem Einschieben der zweiten Schraubwendel 11 der erste Gewebelappen 5 mit Hilfe des ersten Schraubkatheters 8 und dessen Schraubwendel 9 an einem Ausweichen dadurch gehindert wird, dass auf diesen ersten Schraubkatheter 8 eine entsprechende Zugkraft ausgeübt wird.

Eine abgewandelte Ausführungsform zeigt Fig.7 und 8, wobei auch für diese Ausführungsform die Darstellung der Fig.3 und 4 zutreffend ist, weil auch zu dieser zweiten Ausführungsform ein erster Schraubkatheter 8 mit Schraubwendel 9 und ein Punktionsinstrument 12 mit Dilatator gehören.

Als Zugkatheter 10 ist dabei ein Saugröhrchen oder -schlauch vorgesehen, der nach dem Punktieren durch den ersten Schraubkatheter 8 und dessen Schraubwendel 9 sowie durch die Punktionsöffnung in dem dem Zuführkatheter 7 zugewandten ersten Gewebelappen 5 hindurch bis zu dem diesen überlappenden zweiten Gewebelappen 6 verschiebbar und mit seinem proximalen Ende in nicht näher dargestellter Weise an eine Unterdruckquelle anschließbar ist, so dass aufgrund dieses Unterdrucks durch eine Zugkraft auf diesen Saugschlauch der hintere zweite Gewebelappen 6 in Berührung mit dem vorderen ersten Gewebelappen 5 gezogen werden kann, wie es in Fig. 8 dargestellt ist. Dabei ist dieses als Zugkatheter 10 dienende Saugröhrchen wiederum als Gegenelektrode ausgebildet und an eine Hochfrequenzspannungsquelle oder einen Hochfrequenzgenerator anschließbar, so dass auch bei diesem Ausführungsbeispiel zwischen dem Zugkatheter 10 und dem Schraubkatheter 8 ein Hochfrequenzstrom zum Koagulieren und Verbinden der beiden Gewebelappen 5 und 6 fließen kann.

Dieses gegenseitige Verbinden der beiden Gewebelappen 5 und 6 gemäß Fig.6 und 8 kann dabei je nach Größe der Öffnung 2 mehrfach nebeneinander vorgenommen werden. Es kann aber auch eine Vorrichtung 1 vorgesehen werden, die mehrere Zuführkatheter 7 mit an einen Hochfrequenzgenerator anschließbarem Hochfrequenzkatheter und Gegenelektrode aufweist.

Bei beiden Ausführungsbeispielen einer Vorrichtung 1 für einen PFO-Verschluss mittels Hochfrequenzstrom-Erwärmung wird zunächst der vordere, dem Zuführkatheter 7 näherliegende erste Gewebelappen 5 mit Hilfe des ersten Schraubkatheters 8 und seiner Schraubwendel 9 fixiert. Danach wird dieser erste Gewebelappen 5 etwa im Zentrum der ihn durchsetzenden Schraubwendel 9 punktiert, also durchlöchert, so dass ein Zugkatheter 10 hindurchgeschoben und in Verbindung mit dem dahinter befindlichen zweiten Gewebelappen 6 gebracht werden kann, wobei die beiden Katheter an einen Hochfrequenz-generator anschließbar sind, so dass zwischen ihnen bei entsprechender Isolierung ein Hochfrequenzstrom über die beiden Gewebelappen 5 und 6 fließen und diese bei gegenseitiger Berührung durch Koagulation "zusammenkleben" kann. Diese gegenseitige Berührung kann mit Hilfe einer zweiten Schraubwendel 11 eines zweiten Schraubkatheters oder mit Hilfe eines Saugröhrchens und eine daran anbringbare Zugkraft bewirkt werden.

Die Vorrichtung 1 zum Verschließen einer durch zwei sich überlappende Gewebelappen oder Hautfalten 5 und 6 im Herzen 3 gebildeten Öffnung 2 (PFO-Verschluss) weist einen transvenös in das Herz 3 einführbaren Zuführkatheter 7 zum transvenösen Einführen einer Einrichtung auf, mit welcher die beiden Gewebelappen 5 und 6 miteinander verbindbar sind. Diese Einrichtung umfasst im wesentlichen einen in dem Zuführkatheter 7 vorschiebbaren und an dessen distalem Ende ausschiebbaren ersten Schraubkatheter 8 mit einer am distalen Ende angeordneten, in den dem Zuführkatheter 7 in Gebrauchsstellung näherliegenden ersten Gewebelappen 5 eindrehbaren Schraubwendel 9 und einen durch den dem Führungskatheter 7 näherliegenden ersten Gewebelappen 5 nach dessen Punktion hindurch mit dem dahinterliegenden zweiten Gewebelappen 6 verbindbaren Zugkatheter 10 auf, der ein zweiter Schraubkatheter mit Schraubwendel 11 oder ein Saugröhrchen sein kann. Damit kann der hintere zweite Gewebelappen 6 an den ersten Gewebelappen 5 bis zur gegenseitigen Berührung herangezogen werden. Ein mit dem ersten Gewebelappen 5 in Berührung befindlicher Hochfrequenzkatheter kann mit einem Pol und der zum Heranziehen des zweiten Gewebelappens 6 dienende Zugkatheter 10 als Gegenelektrode mit einem weiteren Pol versehen sein, wobei zwischen beiden Elektroden oder Polen eine Hochfrequenzspannung anlegbar ist und die beiden Elektroden voneinander in Gebrauchsstellung beabstandet und/oder gegeneinander isoliert sind, um eine Koagulation der Berührstelle der beiden Gewebelappen 5 und 6 zu ermöglichen.

Es sei noch erwähnt, dass der erste Schraubkatheter 8 mit seiner Schraubwendel 9 nach dem Heranziehen des zweiten Gewebelappens 6 an den ersten Gewebelappen 5 entfernbar und durch einen eigenständigen Hochfrequenzkatheter ersetzbar sein kann, der dann mit dem an dem Zugkatheter 10 befindlichen Pol in gleicher Weise zusammenwirken und eine Koagulation bewirken kann. Bei einer solchen nicht dargestellten Ausführungsform braucht der erste Schraubkatheter 8 nicht elektrisch leitfähig und/oder isoliert und mit einer Anschlussvorrichtung zu einem Hochfrequenzgenerator versehen zu sein. Vielmehr kann nachträglich ein Hochfrequenzgenerator über den Zugkatheter, der ihn dabei führen kann, soweit durch den Zuführkatheter 7 vorgeschoben werden, bis er den ersten Gewebelappen 5 berührt.

## Patentansprüche

1. Vorrichtung (1) zum Verschließen einer durch zwei sich überlappende Gewebelappen (5, 6) im Herzen (3) gebildeten Öffnung (2) in einer im Herzen (3) zwischen zwei Herzkammern, insbesondere zwischen dem rechten Vorhof und dem linken Vorhof, befindlichen Trennwand, mit einem transvenös in das Herz (3) einführbaren Zuführkatheter (7) zum transvenösen Einführen einer Einrichtung zum Verbinden der Gewebelappen (5, 6) miteinander, mit einem durch den dem Zuführkatheter (7) näherliegenden ersten Gewebelappen (5) hindurch mit dem dahinterliegenden zweiten Gewebelappen (6) verbindbaren Zugkatheter (10), mit welchem der dem Zuführkatheter (7) abgewandte zweite Gewebelappen (6) an den ersten Gewebelappen (5) bis zur gegenseitigen Berührung heranziehbar ist, wobei ein mit dem ersten Gewebelappen (5) in Berührung befindlicher Hochfrequenzkatheter mit einem Pol und der zum Heranziehen des zweiten Gewebelappens (6) dienende Zugkatheter (10) als Gegenelektrode mit einem weiteren Pol ausgebildet sind und zwischen beiden Elektroden oder Polen eine Hochfrequenzspannung anlegbar ist und die beiden Elektroden voneinander beabstandet und/oder gegeneinander isoliert sind, wobei die Einrichtung zum Verbinden einen in dem Zuführkatheter (7) verschiebbaren und an dessen distalem Ende ausschiebbaren ersten Schraubkatheter (8) aufweist, der mit dem ersten Gewebelappen (5) in Berührung bringbar ist und durch welchen der Zugkatheter (10) hindurch verschiebbar ist, **dadurch gekennzeichnet, dass** der erste Schraubkatether (8) wenigstens eine am distalen Ende angeordnete, in den den Zuführkatheter (7) in Gebrauchsstellung näherliegenden ersten Gewebelappen (5) eindrehbare Schraubwendel (9) aufweist und dass der zum Heranziehen des zweiten Gewebelappens (6) an den ersten Gewebelappen (5) dienende Zugkatheter (10) als zweiter Schraubkatheter mit wenigstens einer Schraubwendel (11) ausgebildet ist, die zusammen mit ihm durch den ersten Schraubkatheter (8) hindurch verschiebbar ist und deren Schraubwendel oder Schraubwendeln (11) in den den Zuführkatheter (7) ablegenden zweiten Gewebelappen (6) eindrehbar ist oder sind, oder dass als Zugkatheter (10) ein Saugröhrchen oder -schlauch vorgesehen ist, der durch eine Punktionsöffnung in dem dem Zuführkatheter (7) zugewandten ersten Gewebelappen (5) bis zu dem diesen überlappenden zweiten Gewebelappen (6) verschiebbar und mit seinem proximalen Ende an eine Unterdruckquelle anschließbar ist.

2. Vorrichtung nach Anspruch 1, **dadurch gekennzeichnet, dass** der erste Schraubkatheter (8) als Hochfrequenzkatheter ausgebildet und mit einem Hochfrequenzspannungsgeber oder Hochfrequenz-Generator verbindbar ist.

3. Vorrichtung nach einem der Ansprüche 1 oder 2, **dadurch gekennzeichnet, dass** sie ein Punktionsinstrument (12) und/oder einen Dilatator aufweist, welches Instrument durch den Zuführkatheter (7) und/oder durch den ersten Schraubkatheter (8) hindurch verschiebbar und zur Bildung einer Durchtrittsöffnung im inneren Bereich der Schraubwendel (9) des ersten Schraubkatheters (8) durch den ersten Gewebelappen (5) hindurch stechbar ist.

## Claims

1. Device (1) for closing an opening (2), formed by two overlapping flaps of tissue (5, 6) in the heart (3), in a separating wall located in the heart (3) between two chambers of the heart, particularly between the right atrium and the left atrium, having a delivery catheter (7) that can be inserted transvenously into the heart (3) for the transvenous introduction of a device for attaching the tissue flaps (5, 6) to each other, having a tension catheter (10) that can be connected, through the first tissue flap (5) that is closer to the delivery catheter (7), to the second tissue flap (6) located behind it, said tension catheter (10) serving to pull the second tissue flap (6) which is remote from the delivery catheter (7) towards the first tissue flap (5) until they are in mutual contact, while a high frequency catheter that is in contact with the first tissue flap (5) is formed with one pole and the tension catheter (10) serving to pull the second tissue flap (6) towards it is formed as a counter-electrode with another pole, and a high frequency voltage can be applied between the two electrodes or poles and the two electrodes are spaced apart and/or insulated from one another, while the attachment device comprises a first screw catheter (8) that is movable within the delivery catheter (7) and can be pushed out of the distal end thereof, and can be brought into contact with the first tissue flap (5) and through which the tension catheter (10) can be pushed,
**characterised in that** the first screw catheter (8) has at least one screw helix (9) arranged at the distal end that can be rotated into the first tissue flap (5) located closer to the delivery catheter (7) in the position of use, and **in that** the tension catheter (10) serving to bring the second tissue flap (6) close to the first tissue flap (5) is constructed as a second screw catheter with at least one screw helix (11) which can be moved through the first screw catheter (8) therewith, and the screw helix or helices (11) of which can be rotated into the second tissue flap (6) remote from the delivery catheter (7), or **in that** there is provided, as the tension catheter (10), a suction pipe or tube which is movable through a puncture opening in the first tissue flap (5) facing the delivery catheter (7) up to the second tissue flap (6) overlapping it, and can be connected by its proximal end to a vacuum source.

2. Device according to claim 1, **characterised in that** the first screw catheter (8) is constructed as a high frequency catheter and can be connected to a high-frequency voltage source or high frequency generator.

3. Device according to one of claims 1 or 2, **characterised in that** it comprises a puncture instrument (12) and/or a dilator, this instrument being capable of being passed through the delivery catheter (7) and/or through the first screw catheter (8) and being able to be pushed through the first tissue flap (5) to form a through-opening in the inner region of the screw helix (9) of the first screw catheter (8).

## Revendications

1. Dispositif (1) pour fermer une ouverture (2) formée dans le coeur (3) dans une cloison située dans le coeur (3) entre deux chambres cardiaques, en particulier entre l'oreillette droite et l'oreillette gauche, par deux lambeaux de tissu (5, 6) se chevauchant, avec un cathéter d'amenée (7) pouvant être introduit dans le coeur (3) par voie transveineuse pour introduire par voie transveineuse un dispositif destiné à relier ensemble les lambeaux de tissu (5, 6), avec un cathéter de traction (10) pouvant être relié à travers le premier lambeau de tissu (5) le plus proche du cathéter d'amenée (7) au deuxième lambeau de tissu (6) situé derrière, avec lequel le deuxième lambeau de tissu (6) éloigné du cathéter d'amenée (7) peut être rapproché du premier lambeau de tissu (5) jusqu'au contact mutuel, un cathéter à haute fréquence en contact avec le premier lambeau de tissu (5) étant conformé avec un pôle et le cathéter de traction (10) servant au rapprochement du deuxième lambeau de tissu (6) étant conformé en contre-électrode avec un autre pôle et une tension à haute fréquence pouvant être appliquée entre les deux électrodes ou pôles et les deux électrodes étant distantes l'une de l'autre et/ou isolées l'une par rapport à l'autre, le dispositif destiné à relier présentant un premier cathéter fileté (8) qui peut coulisser dans le cathéter d'amenée (7) et sortir à son extrémité distale, qui peut être amené en contact avec le premier lambeau de tissu (5) et à travers lequel le cathéter de traction (10) peut coulisser, **caractérisé en ce que** le premier cathéter fileté (8) présente au moins un filament hélicoïdal (9) disposé à l'extrémité distale, pouvant être vissé dans le premier lambeau de tissu (5) le plus proche du cathéter d'amenée (7) en position d'utilisation, et que le cathéter de traction (10) servant au rapprochement du deuxième lambeau de tissu (6) contre le premier lambeau de tissu (5) est conformé en deuxième cathéter fileté avec au moins un filament hélicoïdal (11) qui peut coulisser conjointement avec lui à travers le premier cathéter fileté (8) et dont le filament hélicoïdal ou les filaments hélicoïdaux (11) peut ou peuvent être vissé(s) dans le deuxième lambeau de tissu (6) éloigné du cathéter d'amenée (7), ou qu'il est prévu comme cathéter de traction (10) un petit tube ou tuyau d'aspiration qui peut coulisser à travers une ouverture de ponction dans le premier lambeau de tissu (5) tourné vers le cathéter d'amenée (7) jusqu'au deuxième lambeau de tissu (6) en chevauchement avec le premier et qui peut être raccordé à une source de dépression à son extrémité proximale.

2. Dispositif selon la revendication 1, **caractérisé en ce que** le premier cathéter fileté (8) est conformé en cathéter à haute fréquence et peut être relié à une source de tension à haute fréquence ou un générateur à haute fréquence.

3. Dispositif selon l'une des revendications 1 ou 2, **caractérisé en ce qu'**il présente un instrument de ponction (12) et/ou un dilatateur, lequel instrument peut coulisser à travers le cathéter d'amenée (7) et/ou à travers le premier cathéter fileté (8) et peut perforer le premier lambeau de tissu (5) pour former une ouverture de passage dans la région intérieure du filament hélicoïdal (9) du premier cathéter fileté (8).
